# NEW EUROPEAN PATENT SPECIFICATION

(11) **EP 1 307 171 B2**
(45) Date of publication and mention of the opposition decision: **07.01.2015**
(45) Mention of the grant of the patent: 20.12.2006
(21) Application number: 01962033.5
(22) Date of filing: 09.08.2001
(51) Int. Cl.: A61K 6/083

(54) **DENTAL COMPOSITIONS COMPRISING BISACRYLAMIDES AND USE THEREOF**
BISACRYLAMID ENTHALTENDE DENTALMASSEN UND DEREN VERWENDUNG
COMPOSITIONS DENTAIRES CONTENANT DES BISACRYLAMIDES ET UTILISATION CORRESPONDANTE

(30) Priority: 11.08.2000 US 224670 P; 07.08.2001 US 310572 P
(43) Date of publication of application: 07.05.2003
(60) Divisional application: 04000245.3
(73) Proprietor: DENTSPLY International Inc., York, PA 17405-0872 (US)
(72) Inventor: WALZ, Uwe, 78465 Konstanz (DE); KLEE, Joachim, E., 78315 Radolfzell (DE)
(74) Representative: Hartz, Nikolai
(86) International application number: PCT/US2001/025032
(87) International publication number: WO 2002/013768

(56) References cited:
- EP-A2- 1 222 910
- WO-A-92/21314
- WO-A-93/12759
- WO-A1-99/03444
- DE-A- 2 211 128
- US-A- 5 545 676
- PATENT ABSTRACTS OF JAPAN vol. 010, no. 262 (C-371), 6 September 1986 (1986-09-06) & JP 61 087608 A (GIICHI TADA;OTHERS: 01), 6 May 1986 (1986-05-06)
- FERRUTI P ET AL: "RECENT RESULTS ON FUNCTIONAL POLYMERS AND MACROMONOMERS OF INTERESTAS BIOMATERIALS OR FOR BIOMATERIAL MODIFICATION" BIOMATERIALS, ELSEVIER SCIENCE PUBLISHERS BV., BARKING, GB, vol. 15, no. 15, 1994, pages 1235-1241, XP001053477 ISSN: 0142-9612
- HILL I R C ET AL: "In vitro cytotoxicity of poly(amidoamine)s: relevance to DNA delivery" BBA - GENERAL SUBJECTS, ELSEVIER SCIENCE PUBLISHERS, NL, vol. 1427, no. 2, 19 April 1999 (1999-04-19), pages 161-174, XP004276296 ISSN: 0304-4165

## Description

### Technical background

Since decades the free-radical polymerization used in electrotechnics, electronics, dental industry, is combined with remarkable advantages in these fields. The frequently used acrylates and methacrylates are applied in combination with pigments and fillers or as pure polymerizable resins. It is well-known that during free-radical polymerization some side-reactions take place. One of them is the inhibition of the outer layer of the polymerizable material due to the influence of oxygen. The thickness of this layer depends on the viscosity of the polymerizable material, the degree of filling, the applied temperature and the time of polymerization. Frequently, the oxygen inhibited layer is disadvantageous due to the mechanical properties in this part are insufficient, the abrasion is higher and the toxicological/allergic potential is increased. The polymerization of very small layers is limited due to the oxygen inhibition, for example in case of covering electronic circuits by screen printing or for dental sealing materials or varnishes.

Furthermore, the conventional methacrylates that were used for dental applications are ester compound. Consequently, they hydrolysis under acidic or basic conditions that frequently leads to a long-term failure.

In order to reduce the oxygen inhibited layer different possibilities were suggested. One of them is the today well-known use of carbonyl/amine initiator systems for photochemical polymerization (R.S. Davison, J.W. Goodin, Eur.Polym.J 18 (1982) 597). Dekker used special color initiators that change triplet-oxygen into singulet-oxygen (C. Dekker, Makromol. Chem. 180 (1979) 2027). Furthermore, surface active additives were used (C.R. Morgan, A.D. Ketley, J. Radiat.Curing7 (1980) 10) or the photochemical SH-En-Addition was applied (C.R. Morgan, F. Magnotta, A.D. Ketley, J.Polym.Sci., Polym. Ed. 15 (1977), 627).

The photochemical polymerization of monoacrylamides was studied by Smets (G. Smets, Bull. Soc. Chim. Belges 71 (1962) 857, G. Oster, J.Amer.Chem.Soc. 79 (1957) 595). A large number of bisacrylamides were described by Ferrutti (P. Ferrutti et al., Polymer 26 (1985) 1336). These bisacrylamides are solids that are soluble in water due to the secondary amide group or they comprises a piperidine group.

A combination of free-radical and Michael addition polymerization was suggest for encapsulation of electronic circuits (DD 295645; invs.: J. Klee, H.-H. Hörhold, I. Scherlitz-Hofmann).

The new synthesized bisacrylamides should be liquids in order to polymerized them without of solvents and furthermore they and the resulting polymers should be insoluble in water.

### Description of the invention

The present invention provides a dental composition according to claim 1. A dental composition that comprises at least one acrylamide selected from bisacrylamide, polyacrylamide, bis(meth)acrylamide and poly(meth)acrylamide; a polymerizable monomer; at least one amine and/or an initiator; a stabilizer; pigments and an organic and/or inorganic filler and that have an improved hydrolysis stability.

The bisacrylamide are characterized by the following formula: 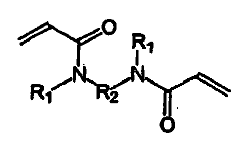 wherein,
the bisacrylamides are characterized by the following formula: 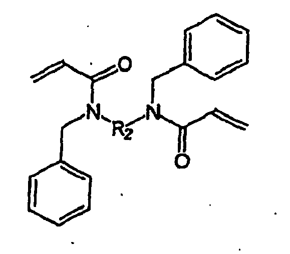 wherein
- R₂: is a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene,

The claimed dental composition preferably contains as polymerizable monomer a mono- or a polyfuncbonal (meth)-acrylate, such as a polyalkylenoxide di- and poly-(meth)acrylate, an urethane di- and poly(meth) acrylate, a vinyl-, vinylen- or vinyliden-, acrylate- or methacrylate; preferably were used diethyleneglycol dimethacrylate, triethyleneglycol dimethacrylate, 3,(4),8,(9)-dimethacryloyloxymethyltricyclodecane, dioxolan bismethacry-fate, glycerol trimethacrylate, furfuryl methacrylate or a monoacrylamide in a content of 5 to 80 wt-%.

Bisacrylamides react with amines in a thermal Michael addition polymerization.

Furthermore the claimed dental composition can contain a polymerization initiator, that preferably is a thermal initiator, a redox-initiator or a photo initiator such as champhor quinone.

In order to avoid a spontaneous polymerization stabilizer may be added such as a radical absorbing monomer for example hydrochinonmonomethylether, hydrochinondimethylether, 2,6-di-tert.-butyl-p-cresol.

The dental composition comprises an inorganic filler and/or an organic filler. Preferably inorganic fillers such as La₂O₃, ZrO₂, BiPO₄, CaWO₄, BaWO₄, SrF₂, Bi₂O₃, glasses or an organic fillers, such as polymer granulate or a combination of organic/or inorganic fillers are applied.

The dental composition is preferably usable as dental root canal filling material or as pulp capping material.

### Example 1 (REFERENCE)

N,N'-bisacryloyl-N,N'-dibenzyl-5-oxanonanediamine-1,9: In a 4-necked 1-I-flask equipped with a stirrer, a thermometer and two 50 ml dropping funnels 102.16 g (0.3 mol) of N,N'-dibenzyl-5-oxanonanediamine-1.9 were dissolved in 300 ml of methylenechloride. After cooling to 0-5 °C 57.020 g (0.63 mol) of acryloylchloride dissolved in 30 ml of methylenechloride and 25.20 g (0.63 mol) of NaOH dissolved in 60 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 150 ml of 1 n HCl, 150 ml of 1 n NaHCO₃ and sometimes with 150 ml of deionised water until the water shows a pH-value of approximately 7. Than the organic solution was dried over NaSO₄. Thereafter the NaSO₄ was filtered off and to the solution 0.1346 g of 2,6-di-tert.-butyl-p-cresol were added. The methylenechloride was removed at 40 °C in vacuum and the bisacrylamide was dried.
Yield: 132.6 g (98.5 % of th.), n_{D}²⁰ = 1.5499, η = 2.35 Pa*s, Mₙ (vpo) = 450g/mol

| | | | | |
|---|---|---|---|---|
| C₂₈H₃₆N₂O₃, 448.61 | calc | C 74.97 | H 8.09 | N 6.24 |
| | found | C 74.50 | H 8.09 | N 6.24 |

IR: 1655 cm⁻¹ (CONR), 1620 cm⁻¹ (CH₂=CH-)

| | |
|---|---|
| ¹H-NMR: | 7.4-7.2 (Ph), 6.65/4.52 (CH₂Ph), 5.58/6.38 (CH₂=CH), 3.4-3.2 |
| | (CH₂O, CH₂N), 1.6-1.5 (CH₂CH₂) |
| ¹³C-NMR: | 166.69/166.28 (3), 137.60/136.95 (5), 129.66/128.95 (2), |
| | 128.80/128.50 (6), 128.35/128.23 (7), 128.16/128.00 (8), |
| | 127.27/126.25 (1), 70.40/70.27 (12), 50.99/48.88 (4), |
| | 48.07/46.97 (9), 27.43/27.11 (11), 25.43/23.15 (10) |

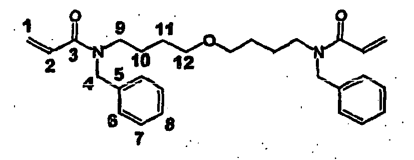

### Addition polymerization:

5.000 g (11.137 mmol) of N,N'-bisacryloyl-N,N'-dibenzyl-5-oxanonanediamine-1.9 and 3.792 g (11.137 mmol) were mixed homogeneously together and reacted for 60 hours at 60 °C. The addition polymer shows the following results obtained by GPC:

| Mₙ | M_{w} | M_{z} | M_{w}/Mₙ | [η] |
|---|---|---|---|---|
| g mol⁻¹ | g mol⁻¹ | g mol⁻¹ | | ml g⁻¹ |
| 3615 | 9403 | 16280 | 2.60 | 8.741 |

### Example 2

N,N'-bisacryloyl-N,N'-dibenzylethylenediamine: In a 4-necked 1-I-flask equipped with a stirrer, a thermometer and two 50 ml dropping funnels 29.198 g (0.12 mol) of N,N'-dibenzylethylenediamine were dissolved in 100 ml of methylenechloride. After cooling to 0-5 °C21.991 g (0.24 mol) of acryloylchloride dissolved in 30 ml of methylenechloride and 9.718 g (0.24 mol) of NaOH dissolved in 40 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 100 ml of deionised water until the water shows a pH-value of approximately 7. Than the organic solution was dried over NaSO₄. Thereafter the NaSO₄ was filtered off and to the solution 0.028 g of 2,6-di-tert.-butyl-p-cresol were added. The methylenechloride was removed at 40 °C in vacuum and the bisacrylamide was dried.
Yield: 27.9 g (65.9 % of th.), mₚ = 75,5-76,6 °C, Tg = -7.2 °C, Mₙ (vpo) = 350g/mol

| | | | | |
|---|---|---|---|---|
| C₂₂H₂₄N₂O₂, 348.45 | calc. | C 75.83 | H 6.94 | N 8.04 |
| | found | C 76.00 | H 7.26 | N 8.05 |

### Example 3 (REFERENCE)

N,N'-bisacryloyl-N,N'-dibenzyl-4,4'-diaminodicyclohexylamine: In a 4-necked 1-I-flask equipped with a stirrer, a thermometer and two 50 ml dropping funnels 60.551 g (0.16 mol) of N,N'-dibenzyl-4,4'-diaminodicyclohexylamine were dissolved in 150 ml of methylenechloride. After cooling to 0-5 °C 28.061 g (0.31 mol) of acryloylchloride dissolved in 30 ml of methylenechloride and 12.401 g (0.31 mol) of NaOH dissolved in 50 ml of water were added simultaneously under stirring during 1.5 hours so that the temperature remains at 0-5 °C. Thereafter the mixture were stirred at room temperature for additional two hours. Than the reaction mixture were hydrolyzed with 500 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 10 ml of deionised water until the water shows a pH-value of approximately 7. Than the organic solution was dried over NaSO₄. Thereafter the NaSO₄ was filtered off and to the solution 0.077 g of 2,6-di-tert.-butyl-p-cresol were added. The methylenechloride was removed at 40 °C in vacuum and the bisacrylamide was dried.
Yield: 54.0 g (69.9 % of th.), Tg = 47.1 °C

### Application Example 1 (Dental root canal sealer) (REFERENCE)

### Bisacrylamide-Paste

5.0000 g of N,N'-bisacryloyl-N,N'-dibenzyl-5-oxanonanediamine-1.9 of example 1, 3.1642 g of Calciumtungstate, 0.7911 g of Zirconiumoxide, 0.0300 g of Aerosil and 0.0100 g of Fe₂O₃ were mixed homogeneously.

### Amine-Paste

1.8962 g of N,N'-dibenzyl-5-oxanonanediamine-1.9, 0.8423 g of 1-Aminoadamantane, 10.9540 g of Calciumtungstate, 2.7385 g of Zirconiumoxide and 0.3353 g of Aerosil were mixed homogeneously.

Immediately before use both pastes were mixed homogeneously in a ratio of 1/1 (v/v) or 1/1.86 (w/w). The material shows an radio-opacity of 11.5 mm/ mm Al.

## Claims

1. A dental composition that comprises
at least a bisacrylamide, a polymerizable monomer, at least an amine and/or an initiator, a stabilizer, pigments and an organic and/or inorganic filler, wherein said bisacrylamide is **characterized by** the following formula: 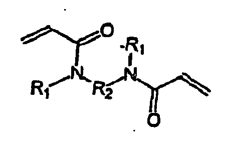 wherein
R₁ is benzyl;
R₂ is a difunctional substituted or unsubstituted C₁ to C₁₈ alkylene, a difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene.

2. Dental composition of claim 1, wherein said polymerizable monomer is a mono- or a polyfunctional (meth)-acrylate, such as a polyalkylenoxide di- and poly- (meth)acrylate,
an urethane di- and poly(meth) acrylate,
a vinyl-, vinylen- or vinyliden-, acrylate- or methacrylate;
preferably diethyleneglycol dimethacrylate, triethyleneglycol dimethacrylate, 3,(4),8,(9)-dimethacryloyloxymethyltricyclodecane, dioxolan bismethacrylate, glycerol trimethacrylate, furfuryl methacrylate or a monoacrylamide in a content of 5 to 80 wt-%.

3. Dental composition of claim 1, wherein said polymerization initiator is a thermal initiator, a redox-initiator or a photo initiator.

4. Dental composition of claim 1, wherein said photo initiator preferably is champhor quinone.

5. Dental composition of claim 1, wherein said filler is an inorganic filler and/or an organic filler.

6. Dental composition of claim 1, wherein said stabilizer is a radical absorbing monomer such as hydrochinonmonomethylether and 2,6-di-tert.-butyl-p-cresol.

## Patentansprüche

1. Dentalmasse, die aufweist:
mindestens ein Bisacrylamid, ein polymerisierbares Monomer, mindestens ein Amin und/oder einen Initiator, einen Stabilisator, Pigmente und einen organischen und/oder anorganischen Füllstoff, worin das Bisacrylamid durch die folgende Formel gekennzeichnet ist: 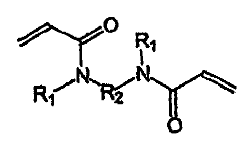 worin bedeuten:
R₁ ist Benzyl,
R₂ ein difunktionelles substituiertes oder unsubstituiertes C₁-C₁₈-Alkylen, difunktionelles substituiertes oder unsubstituiertes Cycloalkylen, difunktionelles substituiertes oder unsubstituiertes C₅-C₁₈-Arylen oder Heteroarylen, difunktionelles substituiertes oder unsubstituiertes C₅-C₁₈-Alkylarylen oder Alkylheteroarylen, difunktionelles substituiertes oder unsubstituiertes C₇-C₃₀-Alkylenarylen.

2. Dentalmasse nach Anspruch 1, worin das polymensierbare Monomer ein mono- oder polyfunktionelles (Meth)acrylat, wie z.B. ein Polyalkylenoxid-di- und - poly(meth)acrylat,
ein Urethan-di- und -poly(meth)acrylat,
ein Vinyl-, Vinylen oder Vinylidenacrylat- oder -methacrylat;
vorzugsweise Diethylenglykoldimethacrylat, Triethylenglykoldimethacrylat, 3,(4),8,(9)-Dimethacryloyoxymethyltricyclodecan, Dioxolanbismethacrylat, Glycerintrimethacrylat, Furfuryl-methacrylat oder ein Monoacrylamid mit einem Gehalt von 5 bis 80 Gew.-% ist.

3. Dentalmasse nach Anspruch 1, worin der Polymerisationsinitiator ein thermischer Initiator, ein Redox-Initiator oder ein Photoinitiator ist.

4. Dentalmasse nach Anspruch 1, worin der Photoinitiator vorzugsweise Campherchinon ist.

5. Dentalmasse nach Anspruch 1, worin der Füllstoff ein anorganischer Füllstoff und/oder ein organischer Füllstoff ist.

6. Dentalmasse nach Anspruch 1, worin der Stabilisator ein Radikal-absorbierendes Monomer, wie z.B. Hydrochinonmonomethylether und 2,6-Di-tert-butyl-p-cresol, ist.

## Revendications

1. Composition dentaire qui comprend
au moins un bisacrylamide, un monomère polymérisable, au moins une amine et/ou un initiateur, un stabilisant, des pigments et une charge organique et/ou inorganique, ledit bisacrylamide étant **caractérisé par** la formule suivante : 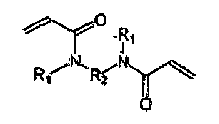 dans laquelle
R₁ représente un groupe benzyle,
R₂ représente un groupe alkylène en C₁ à C₁₈ difonctionnel substitué ou non substitué, cycloalkylène difonctionnel substitué ou non substitué, arylène ou hétéroarylène en C₅ à C₁₈ difonctionnel substitué ou non substitué, alkylarylène ou alkylhétéroarylène en C₅ à C₁₈ difonctionnel substitué ou non substitué, alkylène-arylène en C₇ à C₃₀ difonctionnel substitué ou non substitué.

2. Composition dentaire suivant la revendication 1, dans laquelle ledit monomère polymérisable est
un (méth)acrylate mono- ou polyfonctionnel, tel que les di- et poly(méth)acrylates de poly(oxyde d'alkylène),
les di- et poly(méth)acrylates d'uréthanne,
un acrylate ou méthacrylate de vinyle, vinylène ou vinylidène ;
de préférence le diméthacrylate de diéthylèneglycol, le diméthacrylate de triéthylèneglycol, le 3,(4),8,(9)-diméthacryloyloxyméthyltricyclodécane, le bisméthacrylate de dioxolanne, le triméthacrylate de glycérol, le méthacrylate de furfuryle ou un monoacrylamide en une teneur de 5 à 80 % en poids.

3. Composition dentaire suivant la revendication 1, dans laquelle ledit initiateur de polymérisation est un initiateur thermique, un initiateur redox ou un photo-initiateur.

4. Composition dentaire suivant la revendication 1, dans laquelle ledit photo-initiateur est de préférence la camphoquinone.

5. Composition dentaire suivant la revendication 1, dans laquelle ladite charge est une charge inorganique et/ou une charge organique.

6. Composition dentaire suivant la revendication 1, dans laquelle ledit stabilisant est un monomère absorbant des radicaux, tel que l'éther monométhylique d'hydroquinone et le 2,6-ditertiobutyl-p-crésol.
